# EUROPÄISCHE PATENTSCHRIFT

(11) **EP 1 599 136 B1**
(45) Veröffentlichungstag und Bekanntmachung des Hinweises auf die Patenterteilung: **06.10.2010**
(21) Anmeldenummer: 04716562.6
(22) Anmeldetag: 03.03.2004
(51) Int. Cl.: A61B 5/107

(54) **DREIDIMENSIONALE, DIGITALISIERTE ERFASSUNG DER RAUMFORM VON KÖRPERN UND KÖRPERTEILEN MIT MECHANISCH POSITIONIERTEN BILDGEBENDEN SENSOREN**
THREE-DIMENSIONAL, DIGITIZED CAPTURING OF THE SHAPE OF BODIES AND BODY PARTS USING MECHANICALLY POSITIONED IMAGING SENSORS
DETECTION TRIDIMENSIONNELLE NUMERISEE DE LA FORME DANS L'ESPACE DE CORPS ET DE PARTIES DE CORPS A L'AIDE DE CAPTEURS D'IMAGERIE PLACES DE MANIERE MECANIQUE

(30) Priorität: 05.03.2003 DE 10309788
(43) Veröffentlichungstag der Anmeldung: 30.11.2005
(73) Patentinhaber: corpus.e AG, 70178 Stuttgart (DE)
(72) Erfinder: RUTSCHMANN, Dirk, 70179 Stuttgart (DE)
(74) Vertreter: Prinz & Partner
(86) Internationale Anmeldenummer: PCT/EP2004/002136
(87) Internationale Veröffentlichungsnummer: WO 2004/078040

(56) Entgegenhaltungen:
- EP-A- 0 760 622
- EP-A- 0 958 782
- US-A- 706 459
- US-A- 3 690 242

## Beschreibung

Die Erfassung der Raumform, d.h. der 3-dimensionalen Koordinaten der Oberfläche von Körpern und Körperteilen wie z.B. dem menschlichen Rumpf, der Beinpartien, der Füße usw. zur Auswahl passender Bekleidungen, orthopädischer oder sportlicher Artikel oder zur maßgenauen Herstellung dieser Produkte, zur Gewinnung anatomisch und medizinisch interessierender Daten geschieht heute mit relativ aufwendigen optischen 3D-Scanner, welche üblicherweise auf der Grundlage der Lasertriangulation, der Streifenprojektion oder der Nahbereichs-Photogrammetrie arbeiten. Eine besonders kostengünstige Anordnung eines solchen Scanners ist der sog. "MagicalSkin Scanner" der Fa. corpus.e AG, Stuttgart (www.corpus-e.com). Hierbei wird der zu digitalisierende Körperteil mit einem speziell markierten elastischen, eng anliegenden Überzug bekleidet und mit einer üblichen Digitalkamera freihändig und aus mehreren sich überlappenden Ansichten photographiert. Die photogrammetrische Registrierung, d.h. die Zuordnung korrespondierender Marken aus den einzelnen Bildern erfolgt anhand der speziellen Codierung dieser Marken sowie einer ebenfalls markierten Bodenplatte, auf welcher der zu digitalisierende Kunde steht. Dieses Verfahren ist in einer Reihe von erteilten Patenten und Patentanmeldung beschrieben, so z.B. in dem grundlegenden Patent: EP 0 760 622: Digital Sensing Process and arrangement for the 3-dimensional shape in space of bodies and body parts (Erfinder: Robert Massen).

Die freihändige Positionierung der Digitalkamera um den aufrecht stehenden Kunden herum führt zu einer extrem kostengünstigen Lösung, da keinerlei mechanische Aufbauten, Stative oder kalibrierte Anordnungen von Beleuchtung und Kameras erforderlich sind. Sie erfordert aber eine gewisse Übung in der Handhabung der Kamera, damit sich die einzelnen Bildaufnahmen ausreichend überlappen und die Bildfelder einigermaßen vollständig den zu fotografierenden Körperteil erfassen. Diese Übung ist insbesondere bei Geschäften mit stark wechselndem Personal nicht immer vorhanden. Die falsche Handhabung der Kamera kann zu falschen, d.h. nicht korrekt auswertbaren Aufnahmen führen. Es wäre daher sowohl ein technischer als auch ein wirtschaftlicher Vorteil, wenn unter Beibehaltung der Vorteile dieses extrem kostengünstigen Digitalisierprinzips die Bewegung der Kamera in die verschiedenen, nur ungefähr im Raum definierten Aufnahmepositionen unabhängig von der Geschicklichkeit des Bedienungspersonals durchgeführt werden könnte.

In dem oben genannten Patent wird u.a. eine Anordnung von mehreren fest im Raum positionierten Kameras zur photographischen Aufnahme des Körperteils beschrieben, wobei hierbei als Vorteil die zeitgleiche Aufnahme und die damit kurze Scan-Zeit erwähnt wird. Dieser Vorteil geht allerdings einher mit erhöhten Kosten durch die zahlreichen benötigten Kameras, deren Halterung, und den Verbindungen zum Auswerterechner usw.

Es ist auch bekannt, bei optischen Streifenprojektionssystemen oder Systemen, welche die Silhouette des menschlichen Körpers unter mehreren Ansichten erfassen, den zu digitalisierenden Körper auf eine Drehplatte zu stellen und motorisch in die verschiedenen Aufnahmepositionen zu drehen. Auch dieser Ansatz ist aufwendig, da diese Mechanik das gesamte Körpergewicht tragen muss. Es ist insbesondere auch für ältere Kunden unangenehm, da sie aufrecht und unbeweglich stehen müssen, ohne sich während der motorischen Drehung festhalten zu können.

EP 0 958 782 A1 offenbart eine Vorrichtung und ein Verfahren zum Vermessen von Gliedmaßen. Dabei wird ein Scanner bestehend aus einer Linienlichtquelle und einer CCD-Kamera auf ein Drehgestell montiert und um den zu vermessenden Körper geführt. Der Scanner wirft Licht auf das zu vermessende Objekt und macht aus verschiedenen Winkeln eine Aufnahme des so beleuchteten Objekts. Durch Kenntnis der Kameraposition können die 3D-Koordinaten des Objekts bestimmt werden. Ein Referenzstift erlaubt eine maßstabsgetreue Vermessung.

Es besteht daher ein technisches und wirtschaftliches Interesse daran, über ein kostengünstiges Digitalisiersystem nach Art des MagicalSkin zu verfügen, bei welchem die einzelnen, sich überlappenden Aufnahmen auch durch ein ungeübtes Personal sicher durchgeführt werden können, bei dem der gesamte Körper des Kunden nicht in die einzelnen Aufnahmepositionen bewegt werden muss und bei dem die Kosten des gesamten Systems sehr niedrig bleiben.

Dies wird erfindungsgemäß durch ein Verfahren gemäß Anspruch 1 erreicht. durch Auswertung der photogrammetrischen Marken auf der Unterlage und der photogrammetrischen Marken auf dem Überzug.

Bei Anwendung dieses Verfahrens muss der Patient nicht bewegt werden. Die Führung der Kamera erfolgt auf einer etwa kreisförmigen Bahn, vorgegebene Aufnahmepositionen werden unabhängig vom Bedienungspersonal mechanisch erreicht, so dass immer eine Bildüberlappung sicher gestellt ist, die eine anschließende Auswertung der aufgenommenen photogrammetrischen Marken ermöglicht. Die Ausgestaltung der Sensorführung kann einfach gehalten werden, da bei dem erfindungsgemäßen Verfahren die genaue Position der Kamera, bzw. des bildgebenden Sensors nicht bekannt sein muss. Bei ausreichender Überlappung können mit bekannten Verfahren der Photogrammetrie auf Grund der Marken auf der Unterlage und auf dem zu digitalisierenden Körper die genauen Raumkoordinaten des Körpers oder der Körperteile ohne Kenntnis der exakten Raumposition der Kamera bestimmt werden. Die mechanische Positionierung in vorgegebenen Aufnahmepositionen stellt diese ausreichende Überlappung sicher.

Mit dieser Ausführungsform kann noch flexibler auf unterschiedliche Körper oder Körperteile eingegangen werden, beispielsweise wird, wenn ein Körper überdurchschnittlich viele der auf der Unterlage angebrachten Marken verdeckt, automatisch eine dichtere Bildfolge ausgewertet.

In einer weiteren bevorzugten Ausführungsform kann der bildgebende Sensor zusätzlich zur mechanischen Bewegung um den Körper, den Körperteil oder die Körperteile herum in vertikaler und/oder azimutaler Richtung verstellt werden. Auch diese Ausführungsform erlaubt eine flexiblere Anpassung an verschiedene Körperformen, z.B. wären für Aufnahmen des gesamten Unterkörpers eines Menschen zwei Aufnahmenserien in verschiedenen Höhen denkbar.

Die Erfindung betrifft auch eine Anordnung zur dreidimensionalen, digitalisierten Erfassung der Raumform von Körpern oder Körperteilen gemäß Anspruch 13. durch Auswertung der photogrammetrischen Marken auf der Unterlage und der photogrammetrischen Marken auf dem Überzug.

Ein weiterer bevorzugter Erfindungsgedanke ist, die Kamerahalterung klappbar zu gestalten, so dass zum Transport der herausragende Arm oder Halter auf die markierte Unterlage umgeklappt werden kann.

Ein weiterer bevorzugter Erfindungsgedanke ist, den bildgebenden Sensor auf einer elliptischen Bahn um die zu digitalisierenden Körperteile herum zu fahren. Dies hat insbesondere bei der Aufnahme der Fuß/Bein Partie einer stehenden Person den Vorteil, dass die Abstände von Kamera zu Bein/Fuß in etwa konstant sind.

Die Drehbewegung des Kamerahalters kann durch einen elektrischen Antrieb oder aber auch durch einen mechanischen Antrieb erfolgen. Da das Gewicht des bildgebenden Sensors und der Beleuchtung sehr gering sind, werden hierzu keine großen Kräfte benötigt.

Ein weiterer bevorzugter Erfindungsgedanken ist es, gleichzeitig auf der Kreisbahn mehrere Kameras zu führen, um die Anzahl der Aufnahmepositionen und damit auch die Aufnahmezeit zu verringern.

Ein weiterer bevorzugter Erfindungsgedanke ist, dass auf dem Kamerahalter mehrere Kameras so angebracht sind, dass jede ein anderes Bildfeld mit den gleichen oder mit unterschiedlichen Bildfeldgrößen abdeckt.

Ein weiterer bevorzugter Erfindungsgedanke ist, dass zur optischen Erfassung eines vertikalen Bereiches, welcher größer ist als der aus einer Kameraposition erfassbare, die Kamera kreisförmig um den Kunden in unterschiedlichen Höhen, bezogen auf die Bodenplatte, die Fuß/Bein Partie aufnimmt. Hierzu wird die Kamerahalterung mit einer zusätzlichen vertikalen Linearführung versehen und die Kamera in unterschiedlichen vertikalen Positionen positioniert. Alternativ kann erfindungsgemäß die Kamera in verschiedene Winkelstellungen gebracht werden.

Allen diesen Varianten ist gemeinsam, dass sie mit wenig technischem Aufwand realisiert werden können, da wie bereits oben erwähnt die Kenntnis der genauen Raumposition des bildgebenden Sensors nicht erforderlich ist.

Ein weiterer bevorzugter Erfindungsgedanke ist, dass die Kamera eine Videokamera ist, welche fortlaufend während der mechanischen Bewegung um den Körper herum Bilder aufnimmt und dass mit Verfahren der Mustererkennung automatisch durch Auswertung der Marken der Bodenplatte diejenigen sich teilweise überlappenden einzelnen Bilder ermittelt werden, welche für die photogrammetrische Auswertung erforderlich sind. In der Tat ist es für den Fachmann der Bildverarbeitung und Mustererkennung geläufig, aus den jeweiligen Einzelbildern der Videosequenz den Bereich der Bodenmarken zu extrahieren, diese Marken zu dekodieren und hieraus die Winkelposition der betreffenden Aufnahme zu ermitteln. Die photogrammetrische Auswertung benötigt typischerweise 10 Aufnahmen um den Körper herum, d.h. eine Aufnahme alle 36 Grad. Durch Vergleich der mit Hilfe der Mustererkennung ermittelten Aufnahmepositionen mit dieser Aufnahmevorschrift können alle diejenigen Bilder der Videosequenz gelöscht werden, welche für die photogrammetrische Auswertung nicht benötigt werden. Die Erstellung von Videoaufuahmen im Vergleich zur Einzelbildaufnahme hat den Vorteil, dass die Bildauslösung nur einmal betätigt werden muss, dass die mechanische Bewegung nicht in den einzelnen Aufnahmepositionen gestoppt werden muss und damit insgesamt schneller erfolgen kann.

Ein weiterer bevorzugter Erfindungsgedanke ist, dass jeweils mehrere, senkrecht übereinander angeordnete Bildgeber um den Körper bewegt werden, um auch größere Körper wie z.B. den gesamten aufrecht stehenden Menschen zu erfassen.

Ein weiterer bevorzugter Erfindungsgedanke ist, dass ein Bildgeber mehrfach in unterschiedlichen Höhen, bezogen auf die markierte Unterlage, um den Körper herum bewegt wird um auch größere Körper wie z.B. den gesamten, aufrecht stehenden Menschen zu erfassen.

Ein weiterer bevorzugter Erfindungsgedanke ist, dass innerhalb der Platte, auf welcher der Kunde steht, Sensoren zur Erfassung physikalischer Größen wie Fußdruckmessfelder, Gewichtsmessfelder, Sensoren zur elektroimpedanztechnischen Bestimmung des Körperfettgehaltes und ähnliche, dem Fachmann der Orthopädie und der Medizin bekannte Sensoren angebracht sind. Damit können gleichzeitig zur Digitalisierung der Bein/Fußpartie wichtige anatomische und physiologische Daten des Patienten erfasst werden, welche bei der Auswahl bzw. Maßanfertigung einer Fußbekleidung mit berücksichtigt werden müssen.

Ein weiterer bevorzugter Erfindungsgedanke ist, dass nach der Aufnahme des mit dem elastischen Überzug bekleideten Körpers der Körper unbekleidet in ungefähr dergleichen Stellung aufgenommen wird und damit anatomisch und medizinisch interessierende Aufnahmen erstellt werden.

Ein weiterer bevorzugter Erfindungsgedanken ist, dass diese Aufnahmen des unbekleideten Körpers dem photogrammetrisch erstellten 3D Datensatz in Form einer Textur überlagert werden.

Die beschriebene Anwendung der Fuß/Bein Digitalisierung ist beispielhaft zu verstehen. Der Erfindungsgedanke deckt auch die Digitalisierung anderer Körperpartien wie z.B. des Rumpfes ab. Es ist für den Fachmann ein leichtes, die mechanische Anordnung der Kamera und die zu deren Bewegung erforderliche Führungen und Antrieben entsprechend zu gestalten.

Der Erfindungsgedanke ist keineswegs nur auf die Digitalisierung von menschlichen oder biologischen Körper beschränkt, sondern umfasst alle Objekte, welche zur Digitalisierung mit einem eng anliegenden markierten Überzug bekleidet werden.

Weitere Vorteile und Merkmale der Erfindung ergeben sich aus den Unteransprüchen und aus der folgenden Beschreibung eines Ausführungsbeispiels, nämlich einer Anordnung und einem Verfahren zur 3D-Digitalisierung der Füße von Kunden im Rahmen des Verkaufs passender Fußbekleidungen, mit Bezug auf die Zeichnung, dabei zeigt
- Fig. 1: eine schematische Frontansicht einer erfindungsgemäßen Anordnung zur dreidimensionalen, digitalisierten Erfassung von Unterschenkeln; und
- Fig. 2: eine schematisierte Draufsicht auf eine Unterlage einer erfindungsgemäßen Anordnung.

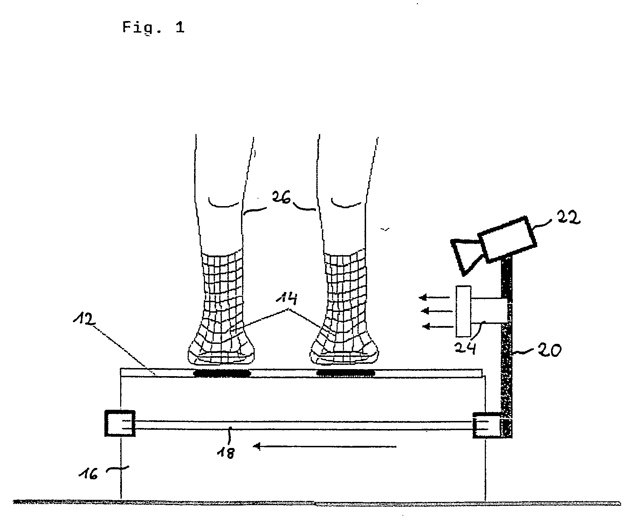

Wie Fig. 1 in einer Frontansicht zeigt, steht ein Kunde aufrecht auf einer mit photogrammetrisch auswertbaren Marken 10 versehenen Unterlage 12 und trägt zwei elastische, eng anliegende Strümpfe 14, welche mit kontrastreichen ebenfalls photogrammetrisch auswertbaren Marken versehen sind. Die Unterlage 12 befindet sich auf einem flachen, kreisförmigen Sockel 16, entlang dessen Umfanges eine umlaufende Führung 18 verläuft. In dieser Führung 18 wird ein senkrechter Halter 20 bewegt, welcher einen bildgebenden Sensor 22 und eine Beleuchtungseinrichtung 24 trägt. Dieser Halter 20 wird motorisch so bewegt, dass sich der bildgebende Sensor 22, beispielsweise eine Digitalkamera, kreisförmig um die beiden Füße/Unterschenkel-Partien 26 des Kunden bewegt. Der Halter 20 mit dem bildgebenden Sensor 22 hält in typischerweise acht bis zwölf vorgewählten, aufeinanderfolgenden Aufnahmepositionen 28 an. Die Stellung des bildgebenden Sensors 22 und die Aufnahmepositionen 28 sind so ausgewählt, dass die von jeder Position aus aufgenommenen Bilder sich überlappende Bildausschnitte aufnehmen, die jeweils die Fuß/Bein-Partie 26 einschließlich zumindest einiger Marken 10 der Unterlage enthalten.

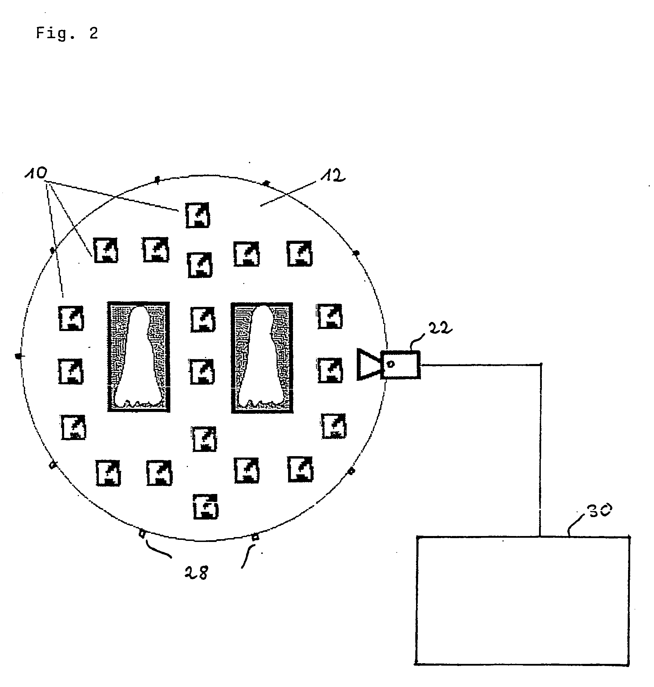

Fig. 2 zeigt eine Draufsicht der Unterlage 12 mit den schematisch angedeuteten Füßen 26 und den photogrammetrisch auswertbaren Marken 10 auf der Unterlage 12. Ebenfalls dargestellt sind der bildgebende Sensor 22 und die nacheinander angefahrenen Aufnahmepositionen 28. Die Kamera ist mit einem Rechner 30 zur Auswertung der aufgenommenen Bilder mit Hilfe von Verfahren der digitalen Bildverarbeitung und der Mustererkennung sowie photogrammetrischer Verfahren verbunden. Es sind zehn Aufnahmepositionen 28 angedeutet, die gleichmäßig um die Unterlage 12 verteilt sind. Je nach aufzunehmendem Körper sind ebenfalls mehr oder weniger oder ungleichmäßig verteilte Aufnahmepositionen 28 denkbar.

Da die photogrammetrische Auswertung dieser sich überlappenden Aufnahmen die Raumposition der einzelnen Kamerapositionen anhand der Marken der Unterlage und der Marken des elastischen Überzugs zurück berechnet, ist keine genaue Kenntnis der jeweiligen Aufnahmeposition erforderlich. Damit kann die Genauigkeit der mechanischen Kameraführung und der einzelnen Aufnahmepositionen sehr gering sein. Die gesamte Mechanik kann einfach, leicht und kostengünstig konstruiert sein. Sie darf sich durchaus unter dem Einfluss des Körpergewichts, von Temperaturschwankungen oder sonstigen Einflüssen verformen, ohne dass darunter die Messgenauigkeit leidet.

## Patentansprüche

1. Verfahren zur dreidimensionalen digitalisierten Erfassung der Raumform von Körpern oder Körperteilen, umfassend die Schritte,
dass der zu digitalisierende Körper, der Körperteil oder die Körperteile (26) mit einem elastischen, eng anliegenden Überzug (14) bekleidet wird oder werden, welcher kontrastreiche, photogrammetrisch auswertbare Marken enthält,
dass der Körper, der Körperteil oder die Körperteile (26) auf eine mit ebenfalls photogrammetrisch auswertbaren Marken (10) versehene Unterlage (12) gestellt wird oder werden,
dass mindestens ein bildgebender Sensor (22) mechanisch auf einer festen Raumbahn um den Körper, den Körperteil oder die Körperteile herum bewegt wird oder werden,
dass in aufeinanderfolgenden Aufnahmepositionen (28), deren Bildausschnitte sich überlappen, jeweils mindestens eine Bildaufnahme erfolgt, welche sowohl den Körper, den Körperteil oder die Körperteile als auch gleichzeitig eine Mehrzahl der photogrammetrisch auswertbaren Marken (10) der Unterlage (12) erfasst,
und dass diese Bildaufnahmen mit Verfahren der Photogrammetrie und der digitalen Bildverarbeitung und Mustererkennung so ausgewertet werden, dass die genauen Raumkoordinaten des aufgenommenen Körpers, des Körperteils oder der Körperteile bestimmt werden, wobei bei der photogrammetrischen Auswertung der sich überlappenden Aufnahmen die Raumposition der einzelnen Aufnahmepositionen des bildgebenden Sensors (22) anhand der Marken der Unterlage und der Marken des elastischen Überzugs zurückberechnet werden.

2. Verfahren nach Anspruch 1, **dadurch gekennzeichnet,**
**dass** der mindestens eine bildgebende Sensor (22) mechanisch auf einer kreisförmigen Raumbahn um den Körper, den Körperteil oder die Körperteile herum bewegt wird.

3. Verfahren nach Anspruch 1, **dadurch gekennzeichnet,**
**dass** der mindestens eine bildgebende Sensor (22) mechanisch auf einer elliptischen Raumbahn um den Körper, den Körperteil oder die Körperteile herum bewegt wird.

4. Verfahren nach einem der vorhergehenden Ansprüche, **dadurch gekennzeichnet,**
**dass** der mindestens eine bildgebende Sensor (22) motorisch bewegt wird.

5. Verfahren nach einem der vorhergehenden Ansprüche, **dadurch gekennzeichnet,**
**dass** der oder die bildgebenden Sensoren (22) nach Art einer Videokamera fortlaufend während einer kontinuierlichen mechanischen Bewegung um den Körper, den Körperteil oder die Körperteile herum Bilder aufnimmt bzw. aufnehmen und
**dass** mit Verfahren der Mustererkennung automatisch durch Auswertung der mitaufgenommenen photogrammetrisch auswertbaren Marken (10) der Unterlage (12) diejenigen, sich teilweise überlappenden einzelnen Bilder ermittelt werden, welche für die photogrammetrische Auswertung ausreichend sind.

6. Verfahren nach einem der vorhergehenden Ansprüche, **dadurch gekennzeichnet,**
**dass** der oder die bildgebenden Sensoren (22) zusätzlich zur mechanischen Bewegung um den Körper, den Körperteil oder die Körperteile herum in vertikaler und/oder azimutaler Richtung verstellt werden kann oder können.

7. Verfahren nach einem der vorhergehenden Ansprüche, **dadurch gekennzeichnet,**
**dass** außerdem Sensoren in der im Kontakt zum Körper, dem Körperteil oder den Körperteilen befindlichen, mit photogrammetrisch auswertbaren Marken (10) versehenen Unterlage (12) physikalische, physiologische, medizinische oder anatomische Größen erfassen.

8. Verfahren nach einem der vorhergehenden Ansprüche, **dadurch gekennzeichnet,**
**dass** der Körper, der Körperteil oder die Körperteile biologische Körper oder Körperteile sind, deren Raumform bestimmt wird, um anatomisch passende Bekleidungen oder orthopädische Hilfsmittel passgenau auszusuchen und/oder herzustellen und/oder um anatomisch und medizinisch interessierende Daten der Raumform zu erfassen.

9. Verfahren nach Anspruch 8, **dadurch gekennzeichnet,**
**dass** die Körperteile die beiden Fuß/Unterschenkel-Partien eines auf einer mit photogrammetrisch auswertbaren Marken (10) versehenen Unterlage (12) stehenden Menschen sind und dass die ermittelten Daten der Raumform zur Auswahl oder zur Maßkonfektion anatomisch passender Fußbekleidung verwendet werden.

10. Verfahren nach Anspruch 8, **dadurch gekennzeichnet,**
**dass** der Körperteil den Unterkörper eines auf einer mit photogrammetrisch auswertbaren Marken (10) versehenen Unterlage (12) stehenden Menschen darstellt und die ermittelten Daten der Raumform zur Auswahl oder zur Maßkonfektion anatomisch passender Kompressionsstrümpfe, passender Strumpfhosen oder passender Hosenbekleidungen verwendet werden.

11. Verfahren nach Anspruch 8, **dadurch gekennzeichnet,**
**dass** nach den Bildaufnahmen des mit dem elastischen Überzug bekleideten Körpers, des Körperteils oder der Körperteile, der gleiche Körper, der Körperteil oder die Körperteile unbekleidet in ungefähr der gleichen Stellung aufgenommen wird/werden.

12. Verfahren nach Anspruch 11, **dadurch gekennzeichnet,**
**dass** die Aufnahmen des unbekleideten Körpers, Körperteils oder der Körperteile dem photogrammetrisch erstellten Raumkoordinaten in Form einer Textur überlagert werden.

13. Anordnung zur dreidimensionalen, digitalisierten Erfassung der Raumform von Körpern oder Körperteilen, umfassend
eine Unterlage (12), die mit kontrastreichen, photogrammetrisch auswertbaren Marken (10) versehen ist und die als Stellfläche dient für den zu digitalisierenden Körper, den Körperteil oder die Körperteile (26), ein elastischer Überzug vorgesehen ist, der ebenfalls photogrammetrisch auswertbare Marken enthält, mit dem der zu digitalisierende Körper, Körperteil oder die Körperteile eng anliegend bekleidet werden kann,
mindestens einen bildgebenden Sensor (22),
mindestens einen annähernd senkrechten Halter (20), an dem der mindestens eine bildgebende Sensor (22) befestigt ist,
einen Sockel (16), auf dem die Unterlage (12) aufliegt.
Mittel zur Bewegung des Halters (20) mit dem bildgebenden Sensor (22) in aufeinanderfolgende Aufnahmepositionen (28), deren Bildausschnitte sich überlappen und gleichzeitig den zu digitalisierenden Körper, Körperteil oder die zu digitalisierenden Körperteile (26) und mehrere der photogrammetrisch auswertbaren Marken (10) der Unterlage (12) erfassen,
Mittel zur Auslösung von Bildaufnahmen in den Aufnahmepositionen (28),
einen Rechner (30), an den die aufgenommenen Bilder übertragen werden können **dadurch gekennzeichnet, dass** der Sockel auf seinem Umfang eine mechanische Führung trägt, entlang welcher der Halter (20) geführt wird und dass der Rechner eingerichtet ist, mit Verfahren der Bildverarbeitung, der Mustererkennung und der Photogrammetrie die Raumform des Körpers, des Körperteils oder der Körperteile zu berechnen, wobei der Rechner weiterhin eingerichtet ist, bei der photogrammetrischen Auswertung der sich überlappenden Aufnahmen die Raumposition der einzelnen Aufnahmepositionen des bildgebenden Sensors (22) anhand der Marken der Unterlage und der Marken des elastischen Überzugs zurück zu rechnen.

14. Anordnung nach Anspruch 13, **dadurch gekennzeichnet**
**dass** der Halter (20) außerdem eine oder mehrere Beleuchtungseinrichtungen (24) trägt.

15. Anordnung nach einem der Ansprüche 13 oder 14, **dadurch gekennzeichnet,**
**dass** der mindestens eine bildgebende Sensor (22) auf dem Halter (20) zusätzlich zur Umfangspositionierung auch in vertikaler und/oder azimutaler Richtung positioniert werden kann.

16. Anordnung nach einem der Ansprüche 13 bis 15, **dadurch gekennzeichnet,**
**dass** zum Transport und zum Verstauen der Anordnung der oder die Halter (20) auf die Ebene der Unterlage (12) geklappt werden kann/können.

17. Anordnung nach einem der Ansprüche 13 bis 16, **dadurch gekennzeichnet,**
**dass** die Mittel zur Bewegung motorische Mittel sind.

18. Anordnung nach Anspruch 17, **dadurch gekennzeichnet,**
**dass** der Sockel (16) sowohl die motorischen Komponenten enthält, welche zur Bewegung des mindestens einen Halters (20) benötigt werden als auch die Rechnerkomponenten (30), welche zur Erfassung der Bilddaten und der Vorverarbeitung und Vorort-Auswertung erforderlich sind.

19. Anordnung nach einem der Ansprüche 13 bis 18, **dadurch gekennzeichnet,**
**dass** die Unterlage (12) Sensoren zur Messung physikalischer, physiologischer, anatomischer oder medizinischer Größen eines zu digitalisierenden Patienten enthält.

## Claims

1. A method for three-dimensional, digitized sensing of the spatial shape of bodies or body parts, comprising the steps of
covering the body, body part or body parts (26) to be digitized with an elastic, tight-fitting cover (14) including high-contrast marks that are photogrammetrically analysable,
placing the body, body part or body parts (26) onto a support (12) which is provided with marks (10) that are also photogrammetrically analysable,
moving at least one imaging sensor (22) mechanically around the body, body part or body parts on a fixed path in space,
taking, in successive shooting positions (28) whose image cutouts overlap each other, at least one respective image which covers both the body, body part or body parts and, simultaneously, a plurality of the marks (10) of the support (12) that are photogrammetrically analysable,
and analysing these image shots by methods of photogrammetry and digital image processing and pattern recognition such that the precise space coordinates of the body, body part or body parts photographed are determined, the photogrammetric analysis of the overlapping shots including back-calculating the position in space of the individual shooting positions of the imaging sensor (22) based on the marks of the support and the marks of the elastic cover.

2. The method according to claim 1, **characterised in**
**that** the at least one imaging sensor (22) is moved mechanically around the body, body part or body parts on a circular path in space.

3. The method according to claim 1, **characterised in**
**that** the at least one imaging sensor (22) is moved mechanically around the body, body part or body parts on an elliptic path in space.

4. The method according to any of the preceding claims, **characterised in**
**that** the at least one imaging sensor (22) is moved by a motor-drive.

5. The method according to any of the preceding claims, **characterised in that** the imaging sensor or sensors (22) continuously take(s) images during a continuous mechanical movement around the body, body part or body parts in the manner of a video camera, and
that by evaluating the simultaneously photographed, photogrammetrically analysable marks (10) of the support (12), using methods of pattern recognition, those individual images, which partially overlap each other, are automatically determined that are sufficient for the photogrammetric evaluation.

6. The method according to any of the preceding claims, **characterised in**
**that** the imaging sensor or sensors (22) can be adjusted in the vertical and/or azimuthal direction in addition to the mechanical movement around the body, body part or body parts.

7. The method according to any of the preceding claims, **characterised in**
**that** in the support (12) which is provided with marks (10) that are photogrammetrically analysable and which is in contact with the body, body part or body parts, sensors further sense physical, physiological, medical or anatomical quantities.

8. The method according to any of the preceding claims, **characterised in**
**that** the body, body part or body parts is/are biological bodies or body parts whose spatial shape is determined in order to choose and/or manufacture anatomically well-fitting clothes or orthopaedic aids to be true to size and/or to acquire anatomically and medically interesting data of the spatial shape.

9. The method according to claim 8, **characterised in**
**that** the body parts are the two foot/lower leg portions of a person standing on a support (12) provided with marks (10) which are photogrammetrically analysable and that the sensed data of the spatial shape are used for the selection or for the custom manufacture of anatomically well-fitting footwear.

10. The method according to claim 8, **characterised in**
**that** the body part constitutes the lower body of a person standing on a support (12) provided with marks (10) which are photogrammetrically analysable and that the sensed data of the spatial shape are used for the selection or for the custom manufacture of anatomically well-fitting compression stockings, well-fitting tights or well-fitting trouser clothes.

11. The method according to claim 8, **characterised in**
**that**, after taking the images of the body, body part or body parts covered with the elastic cover, the same body, body part or body parts is/are photographed in an undressed condition in approximately the same position.

12. The method according to claim 11, **characterised in**
**that** the images of the undressed body, body part or body parts are superimposed on the photogrammetrically established space coordinates in the form of a texture.

13. An arrangement for the three-dimensional, digitized sensing of the spatial shape of bodies or body parts, comprising
a support (12) which is provided with high-contrast, photogrammetrically analysable marks (10) and serves as a floor area for the body, body part or body parts (26) to be digitized, an elastic cover being provided which also includes photogrammetrically analysable marks and with which the body, body part or body parts to be digitized can be covered in a tight-fitting manner,
at least one imaging sensor (22),
at least one approximately vertical holder (20) to which the at least one imaging sensor (22) is attached,
a base (16) on which the support (12) rests,
means for moving the holder (20) together with the imaging sensor (22) to successive shooting positions (28) whose image cutouts overlap each other and simultaneously cover the body, body part or body parts (26) to be digitized and a plurality of the photogrammetrically analysable marks (10) of the support (12),
means for releasing image shots in the shooting positions (28),
a computer (30) to which the images taken can be transmitted, **characterised in that** on its periphery, the base carries a mechanical guide along which the holder (20) is guided, and that the computer is configured to calculate, by methods of image processing, pattern recognition and photogrammetry, the spatial shape of the body, body part or body parts, the computer being further configured to calculate back, in the photogrammetric analysis of the overlapping shots, the position in space of the individual shooting positions of the imaging sensor (22) based on the marks of the support and the marks of the elastic cover.

14. The arrangement according to claim 13, **characterised in**
**that** the holder (20) further carries one or more lighting means (24).

15. The arrangement according to either of claims 13 or 14, **characterised in**
**that** the at least one imaging sensor (22) can also be positioned on the holder (20) in the vertical and/or azimuthal direction in addition to the peripheral positioning.

16. The arrangement according to any of claims 13 to 15, **characterised in**
**that** the holder or holders (20) may be folded onto the plane of the support (12) for transport and for stowing away the arrangement.

17. The arrangement according to any of claims 13 to 16, **characterised in**
**that** the means for moving are motor-driven means.

18. The arrangement according to claim 17, **characterised in**
**that** the base (16) contains both the motor components needed for moving the at least one holder (20) and the computer components (30) required for acquiring the image data and for pre-processing and in situ analysis.

19. The arrangement according to any of claims 13 to 18, **characterised in**
**that** the support (12) contains sensors for measuring physical, physiological, anatomical or medical quantities of a patient to be digitized.

## Revendications

1. Procédé pour la saisie numérisée en trois dimensions de la forme spatiale de corps ou de parties de corps, comprenant les étapes dans lesquelles :
le corps, la partie ou les parties du corps (26) à numériser est ou sont revêtu(e)(s) d'un revêtement (14) élastique moulant qui contient des marques contrastées évaluables par photogrammétrie,
le corps, la partie ou les parties du corps (26) est/sont posé(e)(s) sur un plateau pourvu de marques (10) également évaluables par photogrammétrie,
au moins un capteur d'imagerie (22) est déplacé mécaniquement sur une trajectoire spatiale autour du corps, de la partie du corps ou des parties du corps,
dans des positions de prise de vue (28) successives, dont les découpes d'image se chevauchent, est produite au moins une prise de vue qui saisit tant le corps, la partie du corps ou les parties du corps que, simultanément, une multitude de marques (10) évaluables par photogrammétrie, du plateau (12),
et ces prises de vues sont évaluées avec des procédés de photogrammétrie et de traitement numérique d'imagerie et de reconnaissance de dessins de manière à déterminer les coordonnées spatiales exactes du corps, de la partie du corps ou des parties du corps, la position spatiale des positions de prises de vue individuelles du capteur d'imagerie (22) étant, lors de l'évaluation photogrammétrique des prises de vues se chevauchent, rétrocalculée à l'aide des marques du plateau et des marques du revêtement élastique.

2. Procédé selon la revendication 1, **caractérisé en ce que**
ledit au moins un capteur d'imagerie (22) est déplacé mécaniquement sur une trajectoire spatiale circulaire autour du corps, de la partie du corps ou des parties du corps.

3. Procédé selon la revendication 1, **caractérisé en ce que**
ledit au moins un capteur d'imagerie (22) est déplacé mécaniquement sur une trajectoire spatiale elliptique autour du corps, de la partie du corps ou des parties du corps.

4. Procédé selon l'une quelconque des revendications précédentes, **caractérisé en ce que**
ledit au moins capteur d'imagerie (22) est déplacé par moteur.

5. Procédé selon l'une quelconque des revendications précédentes, **caractérisé en ce que**
le ou les capteurs d'imagerie (22) prend ou prennent des vues à la manière d'une caméra vidéo de façon continue pendant un mouvement mécanique continu autour du corps, de la partie du corps ou des parties du corps, et
avec des procédés de reconnaissance de dessins, on détermine, par évaluation des marques (10) évaluables par photogrammétrie, du plateau (12), les images individuelles se chevauchant partiellement qui sont suffisantes pour l'évaluation photogrammétrique.

6. Procédé selon l'une quelconque des revendications précédentes, **caractérisé en ce que**
le ou les capteurs d'imagerie (22) peut ou peuvent être déplacés en direction verticale et/ou azimutale, additionnellement au mouvement mécanique autour du corps, de la partie du corps ou des parties du corps.

7. Procédé selon l'une quelconque des revendications précédentes, **caractérisé en ce que**
en outre, des capteurs saisissent des grandeurs physiques, physiologiques, médicales ou anatomiques dans le plateau (12) pourvu de marques (10) évaluables par photogrammétrie se trouvant en contact avec le corps, la partie du corps ou les parties du corps.

8. Procédé selon l'une quelconque des revendications précédentes, **caractérisé en ce que**
le corps, la partie du corps ou les parties du corps sont des corps ou des parties du corps biologiques dont la forme spatiale est déterminée pour sélectionner et/ou fabriquer des vêtements anatomiquement ajustés ou des aides orthopédiques et/ou pour saisir des données anatomiquement ou médicalement intéressantes de la forme spatiale.

9. Procédé selon la revendication 8, **caractérisé en ce que**
les parties du corps sont les deux parties pied/jambe d'un homme debout sur un plateau (12) pourvu de marques (10) évaluables par photogrammétrie pour choisir ou pour confectionner sur mesure un revêtement de pied anatomiquement ajusté.

10. Procédé selon la revendication 8, **caractérisé en ce que**
la partie du corps représente la partie inférieure du corps d'un homme debout sur un plateau (12) pourvu de marques (10) évaluables par photogrammétrie pour choisir ou pour confectionner sur mesure des bas de compression anatomiquement ajustés, des collants anatomiquement ajustés ou des pantalons anatomiquement ajustés.

11. Procédé selon la revendication 8, **caractérisé en ce que**
après les prises de vue du corps, de la partie du corps ou des parties du corps revêtu(e)(s) du revêtement élastique, le même corps, la partie du corps ou les parties du corps est/sont filmé(e)(s) sans vêtements dans approximativement la même position.

12. Procédé selon la revendication 11, **caractérisé en ce que**
les prises de vues du corps, de la partie du corps ou des parties du corps sans vêtements sont superposées aux coordonnées spatiales établies par photogrammétrie sous forme d'une texture.

13. Agencement pour la saisie numérisée en trois dimensions de la forme spatiale de corps ou de parties du corps, comprenant
un plateau (12) qui est pourvu de marques contrastées évaluables par photogrammétrie et qui sert d'emplacement pour le corps, la partie du corps ou les parties du corps (26) à numériser, un revêtement élastique qui contient des marques également évaluables par photogrammétrie, avec lequel le corps, la partie du corps ou les parties du corps (26) peut ou peuvent être revêtu(e)(s) d'un vêtement élastique moulant,
au moins un capteur d'imagerie (22),
au moins un support (20) approximativement vertical sur lequel est fixé ledit au moins un capteur d'imagerie (22),
un socle (16) sur lequel repose le plateau (12),
des moyens de déplacement du support (20) avec le capteur d'imagerie (22) dans des positions de prise de vues (28) successives, dont les découpes d'images se chevauchent et saisissent le corps, la partie du corps ou les parties du corps et plusieurs des marques (10) évaluables par photogrammétrie, du plateau (12),
des moyens pour déclencher des prises de vues dans les positions de prises de vues (28),
un ordinateur (30) sur lequel les images filmées peuvent être transmises, **caractérisé en ce que** le socle porte sur sa périphérie un guidage mécanique le long duquel le support (20) est guidé et **en ce que** l'ordinateur est équipé de manière à calculer avec des procédé de traitement d'image, de reconnaissance de dessins et de photogrammétrie la forme spatiale du corps, de la partie du corps ou des parties du corps, l'ordinateur étant en outre équipé pour rétrocalculer, lors de l'évaluation photogrammétrique des prises de vues se chevauchant, la position spatiale des positions de prises de vues individuelles du capteur d'imagerie (22) à l'aide des marques du plateau et des marques du revêtement élastique.

14. Agencement selon la revendication 13, **caractérisé en ce que**
le support (20) porte en outre un ou plusieurs dispositifs d'éclairage (24).

15. Agencement selon l'une des revendications 13 ou 14, **caractérisé en ce que**
additionnellement au positionnement circonférentiel, ledit au moins un capteur d'imagerie (22) peut aussi être positionné en direction verticale et/ou azimutale.

16. Agencement selon l'une des revendications 13 à 15, **caractérisé en ce que**
pour le transport et pour le rangement de l'agencement, le ou les supports (20) peut/peuvent être rabattus sur le plan du plateau (12).

17. Agencement selon l'une des revendications 13 à 16, **caractérisé en ce que**
les moyens de déplacement sont des moyens moteurs.

18. Agencement selon la revendication 17, **caractérisé en ce que**
le socle (16) contient tant les composants moteurs qui sont nécessités pour le déplacement dudit au moins un support (20) que les composants d'ordinateur (30) qui sont nécessaires pour saisir les données d'images et pour le prétraitement et pour l'évaluation sur place.

19. Agencement selon l'une des revendications 13 à 18, **caractérisé en ce que**
le plateau (12) contient des capteurs pour mesurer des grandeurs physiques, physiologiques, anatomiques ou médicales d'un patient à numériser.
